# EUROPEAN PATENT APPLICATION

(11) **EP 2 008 618 A1**
(43) Date of publication of application: **31.12.2008**
(21) Application number: 07111092.8
(22) Date of filing: 26.06.2007
(51) Int. Cl.: A61F 2/34, A61F 2/46

(54) **Prosthesis**

(71) Applicant: Finsbury (Development) Limited, Leatherhead, Surrey KT22 0BA (GB)
(72) Inventor: Tuke, Michael Anthony, Guildford, Surrey GU1 3TF (GB); Taylor, Andrew Clive, Nr Chicester, Sussex PO19 3QQ (GB)
(74) Representative: Smaggasgale, Gillian Helen

(57) **Abstract**

A preassembled unit (1) comprising an acetabular cup prosthesis and a cap (6) covering the cup prosthesis, the cup comprising and outer shell (2) and a liner (3) located within the shell (2).

## Description

The present invention relates to a prosthesis. More particularly, it relates to a preassembled acetabular component for a hip prosthesis.

The efficient functioning of the hip joints is extremely important to the well being and mobility of the human body. Each hip joint is comprised by the upper portion of the femur which terminates in an offset bony neck surmounted by a ball-headed portion which rotates within the acetabulum in the pelvis. Diseases such as rheumatoid- and osteo-arthritis can cause erosion of the cartilage lining of the acetabulum so that the ball of the femur and the hip bone rub together causing pain and further erosion. Bone erosion may cause the bones themselves to attempt to compensate for the erosion which may result in the bone becoming misshapen. This misshapen joint may cause pain and may eventually cease to function altogether.

Operations to replace the hip joint with an artificial implant are well-known and widely practiced. Generally, the hip prosthesis will be formed of two components, namely: an acetabular component which lines the acetabulum; and a femoral component which replaces the femoral head. The femoral component may be total femoral head replacement in which case the component includes a head, neck and a stem which in use in inserted into the end of a prepared femur. Alternatively, where appropriate, the femoral head component may be a resurfacing prosthesis which is attached to the head of the femur once it has been suitably machined.

In an operation to insert a prosthetic acetabulum in a patient's pelvis the surgeon first uses a reamer to grind a cavity of appropriate size in the patient's pelvis. An acetabular cup is then inserted into the cavity. By "appropriate size" is meant a size which is selected by the surgeon as being the most appropriate for that particular patient. Normally, it is desirable to retain as much of the original healthy bone surface as possible.

Commercially available acetabular cups are sold in a range of sizes to suit the needs of individual patients. Generally, acetabular cups are available in sizes of from 42 mm to 62 mm diameter with 2 mm increments between neighboring sizes.

There are a number of different types of prosthetic acetabular cups. One type of cup is those made from polyethylene. There are generally cemented into the acetabulum and require only light pressure to seat them in the cement. One alternative cup type has a polyethylene liner unit for articulation with the femur and a metal shell for insertion into the pelvic cavity. These cups with metal shells may be implanted without cement such that they rely on a jam fit between the metal shell and the patient's acetabulum. Often these metal shells have outer surfaces or coatings which encourage bone to grow into them over time. With this type of prosthesis, the polyethylene liner unit is snapped or screwed into the metal shell after the metal shell has been seated in the acetabulum to form the socket part of the joint.

Prosthetic acetabular cups generally require the use of an insertion tool to achieve correct positioning of the prosthesis in the patient's pelvic cavity. Cups which rely on a jam fit require a greater force to be applied via the insertion tool than is the case with cemented polyethylene cups. This force is usually a direct impact into the acetabulum, but force may also be applied to adjust the angular position of the cup or to remove the cup if it has been positioned incorrectly.

In order that the required forces are accurately and safely applied to the cup, it is necessary that the insertion tool positively grips the cup. However, it is also important that the means by which the tool grips the cup does not impinge upon the outside of the metal shell in order that in use the insertion tool does not become trapped between the shell and the pelvic bone. Further, as the wall thickness of the shell is generally kept to a minimum, the tool cannot generally grip the wall. Insertion tools are therefore generally designed to grip on a mechanical feature provided on the inner hemisphere of the metal shell. This feature is usually designed so as to cause minimum compromise to the function of the prosthetic hip joint. As a result it is often not strong enough for the impaction forces applied which may result in damage to the insertion tool, the meal shell or both.

As acetabular cups are available in a range of sizes, the tools conventionally used to insert them must similarly be provided in a range of sizes such that they can correctly fit and engage with the features provided on the cups. Having to purchase a range of such tools has cost implications for hospitals.

A third category of prosthetic hip joint exists which is manufactured entirely from metal so that the prosthetic articulation comprises a metal on metal joint. These are usually implanted without cement, relying on a jam fit in the acetabulum. With this type of cup the inner hemisphere is not a convenient place to locate a mechanical feature on which the insertion tool could grip. First, the presence of any mechanical feature on the inner surface would reduce the surface area of the prosthetic articulation. Secondly, it could cause damage to the highly polished surface of the metal.

It is therefore desirable to provide an insertion system and in particular an insertion tool for a prosthetic implant in which the attachment means between the insertion tool and the prosthesis is sufficiently robust to withstand the impaction and other forces to which it may be subjected during insertion of the prosthesis and which does not compromise the structural strength or the articulating properties of the prosthesis itself.

One solution to the problems of prior art arrangements is described in GB2323036 in which a prosthetic implant is provided which includes means for attaching a cable to the implant. The cable may secure a cap to the implant. A tool is provided which is connected to the implant by means of the cable. Where the cap is present, the connection of the prosthesis to the tool may be via the cap. In use the surgeon may provide force to the tool to cause the implant to be seated in the bone and then the tool is released. In one arrangement the cable is a continuous cable formed into several loops. Once the prosthesis is located in the desired position, it may be necessary to cut the cable to remove it from the prosthesis.

An alternative arrangement is described in EP1634552 in which a cap is provided for use during insertion of the prosthesis. The cap comprises an impaction plate, at least one cable loop for connecting the impaction plate to a prosthesis, clamping means for attaching the at least one cable means to the impaction plate and cutting means which can be used to sever the at least one loop.

A cap for use to during the insertion of an acetabular cup prosthesis is described in EP1721586. The cap comprises an impaction plate having an upperside and an underside. The impaction plate comprises a plurality of separate sectors mutually connected at a connection point on the impaction plate by means of flanges. Each sector includes interlocking means which in use enable the impaction plate to be connected to an introduction tool. The cap also comprises at least one lug extending downwardly from the underside of each sector. The lug is located at the portion of the underside of the impaction plate which will in use enable the lug to be interconnected with a corresponding recess on the internal wall, an external wall or a rim of a prosthesis.

As indicated above, some acetabular cup prosthesis are used in combination with a liner. Where a liner is to be used, it is usual to implant the metal cup into the pelvis before locating the liner in position in the cup.

Whilst the liner and the cup will generally be manufactured such that the liner is a close fit in the cup, it is difficult for the surgeon to accurately locate the liner. If the liner is incorrectly positioned within the cup by a mere fraction of a millimeter the rim of the liner will not lie flush with the rim of the cup around the entire circumference such that at a part of the cup rim the liner will extend above the cup forming a point at which wear can occur which in use would cause pain to the patient.

Even if the surgeon is able to accurately seat the liner in the cup, there is a risk that during assembly debris may be caught between the liner and the cup.

Generally the surgeon will not insert the shell into the patient before inserting the liner. There is a risk that the insertion of the shell, the walls of which are generally only from about 1mm to about 3mm thick, can become deformed during insertion in view of the forces necessary. If the shell is deformed, it can become difficult or even impossible to insert the liner.

Not only is there a risk that where a portion of the liner stands above the rim of the cup a point of irritation can be produced but in addition, material, such as wear debris, may congregate against the raised portion of liner or against the wall of the cup in the area where the liner sits below the rim. This accumulation of debris may result in a site for post-operative infection.

A further problem which may be encountered is that while inserting the liner it may become damaged. If this damage is a chip or crack on the outer surface of the liner, i.e. on the surface adjacent to the surface of the shell, it may not be noticed. However, its existence will be a point of weakness which can result in the prosthesis failing in use.

Liners may be of any suitable size and may vary in thickness around the liner. However, liners are typically of the order of about 3mm to about 1cm in thickness.

Liners may be prepared from a variety of materials. One example is polyethylene. Although this material has various advantages as being wear resistant, some wear debris may still be released from acetabular cups lined with polyethylene inserts. This debris may cause osteolysis and also trigger adverse reactions in tissue surrounding the hip.

Recently, there has been an increased level of interest in the use of ceramic liners for acetabular cup prosthesis, ceramics have good biocompatibility, hardness, wear resistance and lubrication properties and are therefore potentially very useful materials for use as liners. Although ceramic inserts are useful, their application suffer from the disadvantages detailed above. However, the fragility of the material under the forces provided during insertion mean that they are also difficult to handle.

It is therefore desirable to provide an acetabular component which reduces the risk of liner misplacement and which has enhanced life expectancy arising, in part, through improved resistance to damage caused during impaction into the acetabulum. It is also desirable to provide an acetabular cup prosthesis which can be easily handled and inserted during surgery without damage to the acetabular cup prosthesis and which minimizes the risk of debris being trapped between the cup and the liner.

Thus, according to the present invention, there is provided a preassembled unit comprising an acetabular cup prosthesis and a cap covering the cup prosthesis, the cup comprising:
an outershell; and
a liner located within the shell.

Since the unit is preassembled the liner can be positioned in the outer shell in a controlled manner such as at the manufacturing site. This will enable it's positioning in the cup to be carefully controlled and checked. Further, the liner can be located within the outer shell in a sterile environment thereby minimising the risk of debris being located between the liner and the outer shell of the prosthesis. In addition, the presence of the cap will ensure the integrity of the prosthesis, and keep the cup free of debris, during storage and transit.

Any suitable cap may be used. In one arrangement, the cap comprises an impaction plate; at least one cable loop for connecting the impaction plate to a prosthesis; clamping means for attaching the at least one cable means to the impaction plate; and cutting means for severing the at least one loop at one point along its length.

The impaction plate may be of any suitable configuration but in one arrangement the plate may be configured such that in use it forms a protective covering over substantially the whole of an open face of the acetabular component but does not impinge on the external surface of the cup and therefore does not hamper the insertion of the component into the bone. It will be understood that the term "plate" covers all suitable configurations and may include those which have a differing cross-section through their depth. The shape of the plate will generally be of a substantially disk configuration which may sit at least partly in the cup.

The impaction cap may include a lip on its upper surface which, in use, extends over at least part of an edge of the acetabular component. The lip may be continuous or, in use, it may extend only over one or more portions of the edge of the component. Preferably, the impaction plate extends over at least one or more arcs of the circumference of the rim of the cup.

The impaction plate may be formed from any suitable material. Generally a plastics material such as polyethylene will be used. The material should be suitable to withstand the sterilisation process, be substantially rigid and be able to withstand the impaction forces to which it will be subjected in use.

In one arrangement, the impaction plate may include a neck which extends upwardly from the surface of the impaction plate to surround the clamping and cutting means. The neck may be located centrally of the impaction plate and may surround an aperture in the plate. The neck may be segmented such that arms of the at least one loop may pass between the segments to the clamping means in an arrangement where the clamping means is located in the centre of the neck. The neck may be of a generally frustoconical, annular configuration. The neck may support a platform to which in use an insertion tool may be attached. In arrangements where the gripping tool is attached other than to the platform, the force from the tool may be applied to the platform rather than directly to the surface of the impaction plate.

Whilst the at least one cable loop may extend over the edge of the impaction plate to connect to the acetabular component, the impaction plate will generally include at least one aperture through which the cable passes when forming the at least one cable loop such that the cable can be regarded as passing from the clamping means, through an aperture in the impaction plate, connecting with the acetabular component, returning through the same or a second aperture before returning to the clamping means. It will be understood that this discussion of the cable path is simply to assist understanding and that when the cap used in the present invention is actually being assembled, it may follow the procedure detailed above or an alternative procedure may be used. For example, the at least one cable loop may be preformed and then attached to the prosthesis or they may be formed on the prosthesis and then the free ends passed through apertures on the impaction plate to the clamping means.

The or each apertures in the plate may be slots extending to the edge of the plate or they may be closed apertures such that the cable must be threaded through the aperture. Where there is more than one cable loop present there will generally be at least one aperture associated with each loop.

The or each cable loop will generally connect to the acetabular component by any suitable means. In one arrangement, the component may include a lug around which the cable loop can pass. The lug may extend outwardly from the outer surface of the component or the rim thereof. The lug may extend substantially perpendicularly from the outer wall of the component or may be configured such that it is shaped downwardly to form a cleat under which the cable loop may pass.

In one alternative, a track may be provided in the outer wall of the acetabular component in which the cable may be located. The depth of the track will generally be such that when the cable is in position, it will not be proud of the outer surface of the prosthesis. In one preferred arrangement, the track may be generally arcuate such that the ends of the track open to the rim of the prosthesis. In a further alternative arrangement, a bore in the main body portion of the prosthesis may be provided through which the cable can be threaded.

Where more than one cable loop is provided on the impaction cap, corresponding attachment means will be provided on the acetabular component for each loop. Generally the same kind of attachment means will be provided for each of the loops on an impaction cap where more than one is present.

Where more than one cable loop is used, they may be formed from a single cable such that the cable having returned to the clamping means to form the first loop will then pass through an aperture, be connected to the acetabular component before returning through the same or a further aperture and returning to the clamping means to form a second loop and so on. Alternatively, each loop may be formed from a separate piece of cable, the ends of which will be clamped by clamping means.

Any number of cable loops may be used. However, for the stability of the prosthesis when connected to the cap there will generally be three or more cable loops. Where more than one cable loop is present, they are usually spaced substantially evenly around the cap. Similarly, the corresponding attachment means on the prosthesis will be spaced in a corresponding configuration.

The cable from which the each cable loop is formed may be of any suitable size. The diameter of the cable may be quite small, for example from about 0.5 mm to about 2 mm. It will be understood that where the attachment means of the acetabular component is a bore in the wall of the component, a bore suitable to accept cable of from about 0.5 mm to about 2 mm in the rim of a prosthetic acetabulum of cobalt chrome will not compromise the strength of the implant to any serious degree. Similarly, the provision of lugs having sufficient protrusion to allow a cable of from about 0.5 mm to about 2 mm diameter to be looped therearound on the outer surface of the prosthetic acetabulum, adjacent the rim thereof, will not interfere to any serious degree with the positioning of the acetabular component in the bone cavity. Further a track placed into the outer surface of the wall of the component of the size which will accept cable of this size will not compromise the strength of the component.

The cable from which the at least one loop is formed may be of any suitable material. Suitable materials include metals, ceramics, natural fibers or a plastics material. However, when selecting suitable material, it is necessary to ensure that it has the necessary tensile strength. Where the material is metal, stainless steel is particularly preferred. The cable may be single-stranded but is preferably multi-stranded to provide maximum tensile strength per unit weight of material. In one arrangement, the cable loops may be sheathed in a plastics material such as polypropylene.

It will be understood that while the cable loop will generally have a smooth curved profile, the present invention would operate with a loop which has a substantially rectangular profile.

The clamping means and the cutting means may be separate components or they may be formed from the same component which is capable of carrying out both functions. It will be understood that in one arrangement, the cutting means may not be an integral component of the cap.

In one arrangement the cutting means may be a guillotine arrangement. That is to say it, or a component of the cutting means, will be movable from a raised non-cutting position to a lowered cutting position. The cutting means may be configured such that when the cap used in the present invention is assembled, both arms of at least one the loop pass through the guillotine arrangement. In this configuration, the blade of the guillotine arrangement will be shaped such that it only cuts through one arm so that the other remains hold and held by the clamping means.

The blade may be angled such that it is of generally triangular configuration which slopes from a lowest point located above the arm of the loop which is to be cut to a highest non-cutting side which will be located over the arm of the loop which is not to be cut. In an alternative arrangement, the blade may be stepped such that when it is moved downwardly only one arm is cut. In a further alternative arrangement, whilst a frame holding the blade may move downwardly over both arms, the blade may be provided as a tooth in the frame; the tooth being located over the arm of the loop that is to be cut. However, it will be understood that other cutting means may be used provided that only one arm of the at least one loop is cut.

In an arrangement in which more than one cable loop is present but they are formed out of a continuous cable, one arm of each loop may be severed. However, in one alternative, only one loop will be severed such that the cable can be unthreaded but the two ends remained held by the clamping means.

Although the at least one cable loop will generally be cut through one arm, it will be understood that the cap would be configured to enable the cable to be cut at the head of the loop, although this arrangement is not preferred.

The cutting means may be provided at any suitable position on the cap. For convenience, it may be located in the centre of the impaction plate. Where there is more than one loop present, a cutting means may be provided for each loop. However, generally, a single cutting means will be provided although this will generally include a plurality of blades such that one arm of each loop can be cut. This arrangement may allow one arm of each loop to be cut sequentially or simultaneously.

Any suitable clamping means may be used. In a particularly preferred arrangement, where more than one loop is provided, the clamping means will be located in the centre of the impaction plate such that it can readily retain each loop in place and will take up minimum space on the impaction plate. When the loop as been severed at one point along its length, it will be held by the clamping means such that when the cap is removed from the acetabular component, the cable is removed at the same time.

In a preferred embodiment, the clamping means will be rotatable such that the cable can be wound up into or around the clamping means before the cap is removed from the acetabular component. The clamping means may include a means to enable the user to cause the clamping means to rotate. Thus for example where the clamping means is provided by a nut and bolt arrangement, the user may turn one of the nut or bolt to cause the clamping means to rotate.

In a preferred configuration, the cutting means may be an annular arrangement located around the clamping means. In this arrangement, once the cutting means has been operated, the clamping means may be rotated to draw at least some of the cable into a space between the cutting means and the clamping means.

In an alternative arrangement, the cap may comprise: an impaction plate having an upper side and an underside, the impaction plate comprising a plurality of separate sectors mutually connected with connection points on the impaction plate by means of flanges, each sector including interlocking means which in use enable the impaction plate to be connected to an introduction tool; and at least one lug extending downwardly from the underside of each sector, said lug being located at the portion of the underside of the impaction cap which will in use enable a lug to be interconnected with a corresponding recess in the acetabular cup prosthesis. The recess may be formed in an internal wall, an external wall or a rim of the prosthesis.

The connection point at which the separate sectors are mutually connected is preferably the central point of the impaction plate, A stiffening boss may be provided at the connection point. In one arrangement, the stiffening boss may be located on the undersurface of the cap.

The arrangement of separate sectors mutually connected at a connection point of the impaction plate, means that the cap has some flexibility which enables the cap to be readily connected to, and removed from, the acetabular cup prosthesis this the arrangement allows some flexing of the impact plate. It will be understood that the connection to, and removal from , the acetabular cup prosthesis will involve the insertion of the at least one downwardly extending lug into corresponding depressions in the prosthesis.

The impaction plate may be of any suitable configuration but in one arrangement the sectors will be sized and configured such that in use the impaction plate forms a protective cap over substantially the whole of an open face of the acetabular cup prosthesis but does not impinge on the external surface of the cup and therefore does not hamper the insertion of the prosthesis into the bone. It will be understood that the term "plate" covers all suitable configurations and may include those which have a cross-sectional thickness which varies across the plate. The shape of the plate will generally depend on the specific prosthesis with which the cap is to be used. The impaction plate will generally be of a substantially disk configuration which may sit at least partly in the cup. In a most preferred arrangement, the impaction plate will be circular in shape.

In one arrangement, the edge of the impaction plate will be chamfered. In an alternative arrangement, the impaction plate may include a lip which, in use, extends over at least part of an edge of the prosthesis. The lip may be continuous or, in used, it may extend only over one or more portions of the edge of the prosthesis.

The sectors may be of any suitable configuration. In one arrangement, each sector may be a sector of an annulus. In this arrangement, a flange may extend from a point on the inner radius of the part-annular sector to the connection point. The point from which the flange extends is generally a centre of the inner radius of the part-annular sector.

The impaction plate may include any suitable number of sectors. Generally there will be an even number of sectors. In one preferred arrangement there will be four sectors.

The spacing between the sectors may be the same or different.

In one arrangement of the present invention, each flange connecting a respective sector to the connection point may be configured such that the sectors may move circumferentially. In a preferred embodiment of this arrangement, each flange is configured such that movement of the sectors out of the plane of the cap is minimised. The ability of the sectors to move circumferentially enables the lugs to be readily inserted into the corresponding recesses in an acetabular cup. This arrangement is particularly suitable where the lug is an angled tooth. The circumferential movement allowed by the flange enables the lugs to be snapped into place in corresponding angled recesses in the cup.

In one alternative arrangement, each flange connecting a respective sector to the connection point may be configured such that the sectors may flex out of the plane of the cap. This a particularly preferred arrangement where the lugs are ribs which are snap-fit in corresponding recesses in the cup rim.

In a further alternative arrangement, each flange may be configured so that the sectors may move radially to allow the lugs to be moved between a position in which the lugs engage with corresponding recesses in the cup and a position in which the lugs are disengaged from the corresponding recess.

It will be understood that the, or each, flange may be arranged to allow the, or each, sector to have one or more of the circumferential movement, radial movement out of the plane.

The lugs may be of any suitable arrangement, They will be situated on the underside of the impaction plate at a point that is suitable to enable them to engage corresponding recesses located in the rim of the cup prosthesis. The lugs on the respective sectors may be the same or different.

In one arrangement, the lugs may be ribs. The ribs may extend substantially vertically from the impaction plate or may be angled thereto. In one arrangement, they will be angled such that the ribs slope inwardly. One or more of the ribs may have a dovetail on at least one end thereof.

In an alternative arrangement, the lugs may be configured such that they are shaped as angled teeth. In one arrangement, the angled teeth on adjacent sectors may be angled in opposing directions. These opposing faced teeth in use serve to assist the connection between the cap and the cup.

However configured, the or each lug may be located in the same position on each sector or may be in different positions. They may be equally spaced.

In an alternative arrangement, the cup may be as described in GB2323036.

The impaction plate may be formed from any suitable material. Generally a metal will be used but other materials may be used provided that they have sufficient strength to withstand the impaction and enable the flexibility required for use to be achieved. In addition, the material should be suitable to withstand the sterilisation process.

The interlocking means to enable the impaction plate to be connected to an introducer tool may be any suitable means. In one arrangement, the means are one or more apertures extending through the sector. The one or more apertures may be of any suitable configuration. In one arrangement, the interlocking means may allow the cap to be permanently connected to an introducer tool.

The outer shell of the prosthesis is preferably made from metal. It's outer surface may be configured to promote bone integration. In one arrangement, the outer surface may be coated with, a bone growth promoting material such as hydroxyapatite.

The liner may be formed of any material which has acceptable biocompatibility, hardness and wear resistance. A plastics material such as polyethylene may be used. In one alternative, the liner may be formed from a ceramics material. Suitable ceramic materials include silicon nitride, doped silicon nitride, an alumina-zirconia ceramic, yttria, stabilized zirconia, ceria, stabilized zirconia, zirconia ceramics, alumina ceramics, oxinium or mixtures thereof.

The liner may simply be held in position in the outer cup as a tight fit. Alternatively, the liner may be held in the outer shell of the cup by securement means which are biocompatible and function effectively. Preferred securement means include cement or screws. The securement means may be formed on the inner surface of the outer shell of the cap, on the liner or on both such that the liner on the cap are a snap-fit together.

The present invention will now be described, by way of example, with reference to the following examples in which:

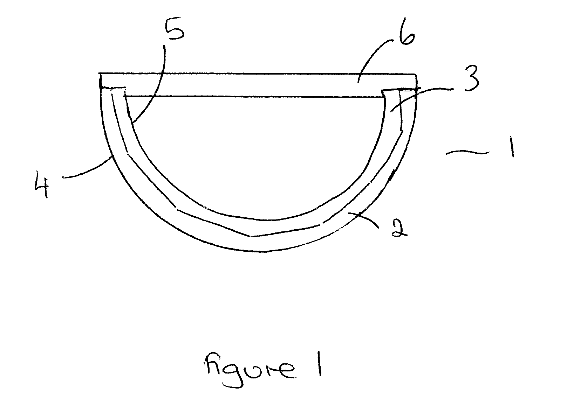

- Figure 1: is a schematic cross-section of one arrangement of the present invention.

As illustrated in Figure 1, the unit 1 of the present invention comprises an acetabular cup prosthesis comprising an outer metal shell 2. It will be understood that the shell 2 has an outer surface 4 which in use is in contact with the acetabulum when implanted therein. The inner surface of shell 2 abuts the outer surface of the liner 3. The outer surface of the liner 3 and the shell 2 are shaped to cooperate as closely as possible to each other and there is a high degree of abutment between them. The inner surface 5 of the liner 3 is the articulating surface for the head, either natural or prosthetic, of the femur.

An impaction cap 6 is connected to the acetabular cup prosthesis. The cap 6 may be of any suitable configuration. The connection between the cap and the cup will depend on the configuration of the cap. Suitable cups include those described in GB2323036, EP1634552 and EP1721586 which are incorporated herein by reference. In addition to protecting the cup prosthesis from ingress of debris in the cup during storage and transport, the cap 6 also serves an important function during insertion in that it spreads impact stress evenly across the cap to ensure that no region of the rim of the cup/liner absorbs an excessive amount of force. This serves to prevent the liner 3 and the cup 2 from being displaced relative to the other and also protects the liner from damage during impaction.

The cap will preferably include means to interconnect with an insertion tool.

Once the prosthesis is in position in the pelvis, the cap 6 is removed and the acetabular cup prosthesis is in position with the rims of the outer shell and liner correctly aligned.

## Claims

1. A preassembled unit comprising an acetabular cup prosthesis and a cap covering the cup prosthesis, the cup comprising and outer shell and a liner located within the shell.

2. A preassembled unit according to Claim 1 wherein the cap comprises:
an impaction plate;
at least one cable loop for connecting the impaction plate to a prosthesis;
clamping means for attaching the at least one cable means to the impaction plate;
and
cutting means for severing the at least one loop at one point along its length.

3. A preassembled unit according to Claim 1 wherein the cap comprises: an impaction plate having an upper side and an underside, the impaction plate comprising a plurality of separate sectors mutually connected at a connection point on the impaction plate by means of flanges, each sector including interlocking means which use enable the impaction plate to be connected to an introduction tool; and at least one lug extending downwardly from the underside of each sector, said lug being located at the portion of the underside of the impaction cap which will in use enable the lug to be interconnected with a corresponding recess on an internal wall, and external wall or the rim of the acetabular cup prosthesis

4. A preassembled unit according to any one of Claims 1 to 3 wherein the outer shell of the cup is a metal cup.

5. A preassembled unit according to any one of Claims 1 to 4 wherein the liner is a plastics material.

6. A preassembled unit according to any one of Claims 1 to 4 wherein the liner is a ceramic material.

7. A preassembled unit according to Claim 6 wherein the ceramic material is alumina ceramic, silicon nitride, zirconia ceramic, oxinium or a mixture thereof.
